# EUROPEAN PATENT SPECIFICATION

(11) **EP 2 123 645 B1**
(45) Date of publication and mention of the grant of the patent: **10.04.2013**
(21) Application number: 08711034.2
(22) Date of filing: 08.02.2008
(51) Int. Cl.: C07D 301/14, C07D 493/08

(54) **OXIDIZING AGENT COMPOSITION FOR THE EPOXIDATION OF OLEFINS AND PROCESS FOR THE EPOXIDATION OF OLEFINS**
OXIDATIONSMITTELZUSAMMENSETZUNG ZUM EPOXIDIEREN VON OLEFINEN UND VERFAHREN ZUM EPOXIDIEREN VON OLEFINEN
COMPOSITION D'AGENT OXYDANT POUR L'ÉPOXYDATION D'OLÉFINES ET PROCÉDÉ POUR L'ÉPOXYDATION DES OLÉFINES

(30) Priority: 22.02.2007 JP 2007042315
(43) Date of publication of application: 25.11.2009
(73) Proprietor: Adeka Corporation, Tokyo 116-8554 (JP)
(72) Inventor: KIKUCHI, Takaaki, Tokyo 116-8554 (JP); NITOH, Hirohisa, Tokyo 116-8554 (JP)
(74) Representative: HOFFMANN EITLE
(86) International application number: PCT/JP2008/052155
(87) International publication number: WO 2008/102659

(56) References cited:
- WO-A1-2004/039791
- JP-A- 03 052 868
- JP-A- 2004 155 635
- JP-A- 2006 299 068
- COOPER M S ET AL: "OXIDATION REACTIONS USING UREA-HYDROGEN PEROXIDE; A SAFE ALTERNATIVE TO ANHYDROUS HYDROGEN PEROXIDE", SYNLETT, GEORG THIEME VERLAG, DE, 1 September 1990 (1990-09-01), pages 533-535, XP001147750, ISSN: 0936-5214, DOI: DOI:10.1055/S-1990-21156
- HARRY HEANEY: "Oxidation Reactions Using Magnesium Monoperphthalate and Urea Hydrogen Peroxide", ALDRICHIMICA ACTA, vol. 26, no. 2, 1993, pages 33-58, XP002646500,
- ASTUDILLO L. ET AL.: 'A Very Simple Oxidation of Olefins and Ketones with UHP-Maleic Anhydride' HETEROCYCLES vol. 36, no. 5, 1993, pages 1075 - 1080, XP002270125
- CHEMICAL ABSTRACTS, vol. 72, no. 25, 1970, Columbus, Ohio, US; abstract no. 131986K, MALINOVSKI M.S. ET AL.: 'Vinylacetylenic acid ester epoxides' page 297; XP008136007 & ZHURNAL ORGANICHESKOI KHIMII vol. 72, 1970, pages 428 - 431
- PIETIKAINEN P. ET AL.: 'Asymmetric Mn(III)-salen catalyzed epoxidation of unfunctionalized alkenes with in situ generated peroxycarboxylic acids' JOURNAL OF MOLECULAR CATALYSIS A: CHEMICAL vol. 165, no. 1-2, 2001, pages 73 - 79, XP008116496
- CHEMICAL ABSTRACTS, vol. 73, no. 10, 1970, Columbus, Ohio, US; abstract no. 46930P, MALINOVSKI M.S. ET AL.: 'Mathematical planning during the epoxidation of unsaturated compounds using urea peroxide for obtaining the maximum epoxy number' page 81; XP008136008 & LAKOKRASOCHNYE MATERIALY I IKH PRIMENENIE vol. 2, 1970, pages 29 - 31
- FAN C.L. ET AL.: 'Epoxidation of Chiral Camphor N-Enoylpyrazolidinones with Methyl (trifluoromethyl)dioxirane and Urea Hydrogen Peroxide/Acid Anhydride: Reversal of Stereoselectivity' THE JOURNAL OF ORGANIC CHEMISTRY vol. 68, 2003, pages 9816 - 9818, XP008116473

## Description

### Technical Field

The present invention relates to a process for epoxidation of olefins using an oxidizing agent composition for epoxidation of olefins, which epoxidizes a double bond contained in the olefins by oxidation.

### Background Art

Epoxy compounds have been used as raw materials in various fields such as in resins, coatings, medicines, agricultural chemicals, and electronic materials, and there are several methods of producing the epoxy compounds. Of those, the method which has been most generally performed is a method involving reacting epichlorohydrin or the like with alcohols (for example, see Patent Document 1). Those epoxy compounds contain impurities, but are cheap and suitable for mass production, and thus are used in many fields. However, all of the halogen atoms derived from the raw material cannot be removed from those epoxy compounds, so there is demand for an epoxy compound which does not contain halogen atoms from the viewpoint of dioxin problems or similar.
Another method, for producing epoxy compound is a method involving the use peroxide compounds such as hydrogen peroxide and peracetic acid (for example, see Patent Documents 2 and 3). When those oxidizing agents are used, the contamination caused by halogen atoms can be avoided, but the yield is poor, and in particular, there is a problem caused by the large amount of glycols being generated as by-products.

Cooper M.S. et al., Synlett, Sept. 1990 relates to oxidation reactions using urea hydrogen peroxide (UHP) as oxidizing agent.

Patent Document 1: JP 05-63250 A
Patent Document 2: JP 05-213919 A
Patent Document 3: JP 06-172335 A

### Disclosure of the Invention

### Problems to be solved by the Invention

An object of the present invention is to provide a process for epoxidation of olefins using an oxidizing agent composition capable of producing a halogen-free epoxy compound in high yield.

### Means for solving the Problems

Through inventive study, the inventors of the present invention have found a process for epoxidation of olefins using an oxidizing agent composition capable of effectively performing such an oxidation reaction, thus, completing the present invention. That is, the present invention provides a process for epoxidation of olefins using an oxidizing agent composition for epoxidation of olefins, characterized by including an acid anhydride and urea peroxide.

### Effects of the Invention

An effect of the present invention is to provide a process for epoxidation of olefins using an oxidizing agent composition capable of producing a halogen-free epoxy compound in high yield. Further, an epoxy compound derived from an alicyclic olefin, the epoxidation of which has conventionally been difficult, can be effectively produced.

### Best Mode for carrying out the Invention

First, acid anhydrides which are used in the present invention are described. The acid anhydrides can be broadly divided into two types in terms of their structures: an acid anhydride obtained by dehydration condensation of two molecules of carbonyl group-containing compounds; and an acid anhydride obtained by intramolecular dehydration condensation of a compound having two or more carbonyl groups in one molecule. These acid anhydrides can be represented by the following general formulae (1) and (2).

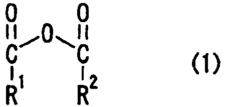

(R¹ and R² each represent a hydrogen atom or a hydrocarbon group which may include an oxygen atom.)

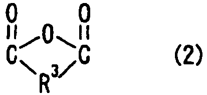

(R³ represents a hydrocarbon group which may include an oxygen atom.)

Examples of the acid anhydride represented by the general formula (1) include acid anhydrides obtained by condensation by dehydration of monocarboxylic acids of the same kind with each other, such as formic acid, acetic acid, propionic acid, butanoic acid (butyric acid), pentanoic acid (valeric acid), isopentanoic acid (isovaleric acid), hexanoic acid (caproic acid), heptanoic acid, isoheptanoic acid, octanoic acid (caprylic acid), 2-ethyl hexanoic acid, isooctanoic acid, nonanoic acid (pelargonic acid), isononanoic acid, decanoic acid (capric acid), isodecanoic acid, undecanoic acid, isoundecanoic acid, dodecanoic acid (lauric acid), isododecanoic acid, tridecanoic acid, isotridecanoic acid, tetradecanoic acid (myristic acid), hexadecanoic acid (palmitic acid), octadecanoic acid (stearic acid), isostearic acid, and oleic acid.

Acid anhydrides, which are used in the present invention, in which the same kind of monocarboxylic acids are condensed by dehydration, are formic anhydride, acetic anhydride, and propionic anhydride. Of those, acetic anhydride is more preferred because it is the most versatile and has high stability. When the molecular weight of the acid anhydride becomes greater, the amount of carboxylic acid per unit weight decreases and the blending amount increases, thus it may be economically disadvantageous. Further, when the molecular weight is large, the removal by water washing is difficult, thus purification after the completion of the reaction may become difficult.

Acid anhydrides, which are used in the present invention, represented by the general formula (2) are succinic anhydride, maleic anhydride, and phthalic anhydride.

Urea peroxide is used in the present invention because of its high effect of epoxidation.

Urea peroxide is produced by using urea and hydrogen peroxide as raw materials, and the production method thereof may involve, for example, adding 1 to 3 mol or preferably 1.1 to 2 mol of hydrogen peroxide with respect to 1 mol of urea, mixing the mixture at 30 to 80°C for 1 to 10 hours, cooling the resultant to deposit crystals of urea peroxide, and separating and drying the crystals. When the content of hydrogen peroxide is less than 1 mol, the reaction rate becomes slow and a large amount of unreacted urea remains in the mixture. Further, when the content of hydrogen peroxide exceeds 3 mol, a large amount of unreacted hydrogen peroxide remains in the mixture. Further, hydrogen peroxide used as the raw material is generally on the market in an aqueous solution form, but when a solution in which the concentration of hydrogen peroxide is low is used, a large amount of water enters into the reaction system and it becomes difficult for urea peroxide to be deposited, therefore, it is preferred that a hydrogen peroxide solution having a high hydrogen peroxide concentration of 50 weight% or more be used.

As for the blending ratio of urea peroxide to the acid anhydride 0.7 to 1.5 mol, or preferably 0.8 to 1.3 mol of urea peroxide is blended with respect to 1 mol of the acid anhydride. When the ratio of the solid hydrogen peroxide adduct to the acid anhydride is too large, there are cases where many by-products are derived from the opening of the generated epoxy rings, and when the ratio is too small, there are cases when the epoxidation reaction does not proceed sufficiently.

The olefins which can be used in the present invention may be terminal olefins or inner olefins as long as the olefins are each an organic compound having a double bond, and the molecule thereof may have one double bond or two or more double bonds. In the present application, in the case where n double bond(s) is/are present in one olefin molecule, it is defined as an n-equivalent olefin. That is, an olefin having one double bond is referred to as a 1-equivalent olefin and the olefin having two double bond is referred to as a 2-equivalent olefin.

Examples of those olefins include: monoolefins such as 1-hexene, 1-decene, cyclohexene, oleyl alcohol, styrene, and allyl alcohol; diolefins such as divinylbenzene, dicyclopentadiene, and limonene; and other trifunctional or higher polyolefins. In general, it is more difficult to epoxidize a compound having two or more double bonds in the molecule compared to a compound having one double bond in the molecule. However, the epoxidation process of the present invention can easily epoxidize a compound without generating by-products, even if the compound is the one having two or more double bonds in the molecule. Further, the epoxidation process of the present invention can easily epoxidize effectively alicyclic olefins such as dicyclopentadiene, the epoxidation of which has conventionally been difficult. Although some of the following examples overlap with the above-mentioned examples of olefins, the alicyclic olefins include cyclohexene, cyclohexadiene, cyclooctene, dicyclopentadiene, α-pinene, limonene, L-carveol, α-terpinene, and β-terpinene.

The oxidation method of the present invention is a method involving mixing the oxidizing agent composition for epoxidation with olefins and epoxidizing the double bonds of the olefins by oxidation. One or more solvents are used during the reaction. However, the mixture is preferably diluted with the solvent and then subjected to the reaction, because there are cases where the reaction does not proceed homogeneously when the reaction system becomes highly viscous or solid. Further the addition of the solvent makes it easier to control reaction heat. Solvents, which are used in the present invention, are selected from the group consisting of methanol, ethanol, propanol, isopropanol, butanol, isobutanol, secondary butanol, tertiary butanol, pentanol, isopentanol, secondary pentanol, neopentanol, tertiary pentanol, hexanol, secondary hexanol, heptanol, secondaryheptanol, octanol, 2-ethylhexanol, benzene, toluene, and xylene, hexane, heptane, decane, and cyclohexane. Of those solvents, benzene, toluene, xylene, hexane, heptane, decane, and cyclohexane are preferred, and toluene, xylene, and hexane are more preferred, because they are easily purified when purification by water washing is conducted after the completion of the reaction.

The process for epoxidzing of the present invention includes: adding, to 1 mol of an n-equivalent olefin (that is, n mol of double bond(s) contained in 1 mol of olefin), 0.5n to 2n mol, preferably 0.8n to 1.8n mol, or more preferably 1.0n to 1.5n mol of each of an acid anhydride and urea peroxide; and allowing the mixture to react preferably by heating the mixture to 20 to 100°C or more preferably 40 to 70°C and preferably stirring the mixture for 1 to 30 hours or more preferably 2 to 15 hours, whereby an epoxy compound is produced. After the completion of the reaction, there remains in the reaction system acid derived from the acid anhydride and urea or the like. Those impurities may be removed by a known purification method such as filtration, water washing, liquid separation, or distillation. Further, the removed acid can be dehydrated and reused as an acid anhydride, and urea or the like can be reacted with hydrogen peroxide to thereby produce urea peroxide and can also be reused.

### Examples

Hereinafter the present invention is described specifically by way of examples. Unless otherwise mentioned, "%" and "ppm" used in examples and the like below are each expressed in terms of weight.

### Example 1

Dicyclopentadiene (2 g: 15.2 mmol) was dissolved in toluene (26.3 g), and a composition containing urea peroxide (3.7 g, 39.4 mmol) and acetic anhydride (3.72 g, 36.5 mmol) was added thereto. The mixture was heated to 60°C and was stirred for 9 hours. After that, the resultant was cooled to 20°C, and the obtained solution was washed three times with 20 ml of pure water, thereby completely removing acetic acid, urea, and hydrogen peroxide remaining in the solution. The resultant was further subjected to distillation thereby removing toluene, whereby 2.42 g (97% yield) of dicyclopentadiene diepoxide was produced.

### Example 2

Dicyclopentadiene (2 g: 15.2 mmol) was dissolved in benzene (30 g), and a composition containing urea peroxide (3.7 g: 39.4 mmol) and phthalic anhydride (7.18 g: 48.5 mmol) was added thereto. The mixture was heated to 60°C and was stirred for 8 hours. After that, the resultant was cooled to 20°C, the deposited phthalic acid was removed by filtration, and the obtained solution was washed three times with 20 ml of pure water, thereby completely removing urea and hydrogen peroxide remaining in the solution. The resultant was further subjected to distillation thereby removing benzene, whereby 2.41 g (97% yield) of dicyclopentadiene diepoxide was produced.

### Comparative Example 1

The reaction was performed in the same manner as the experimental method of Example 1, except that acetic acid (2.19 g: 36.5 mmol) was added instead of acetic anhydride. As a result, the reaction did not proceed at all and 99% of the raw material was collected.

### Comparative Example 2

The reaction was performed in the same manner as the experimental method of Example 1, except that 60% hydrogen peroxide (2.06 g: 36.4 mmol) was added instead of urea peroxide. The yields of dicyclopentadiene diepoxide and dicyclopentadiene diglycol were 75% and 20%, respectively, and 5% of dicyclopentadiene as the raw material was collected.

### Industrial Applicability

By using the present invention, the olefins can be effectively oxidized into the epoxy compounds, and the produced epoxy compounds are free of halogen atoms.
Those epoxy compounds are useful as materials in various technical fields such as resins, coatings, medicines, agricultural chemicals, and electronic materials.

## Claims

1. A process for epoxidizing olefins, comprising reacting an olefin with a mixture obtained by mixing an acid anhydride with urea peroxide in a solvent,
wherein said acid anhydride is one kind or two or more kinds of acid anhydride selected from the group consisting of formic anhydride, acetic anhydride, propionic anhydride, succinic anhydride, maleic anhydride, and phthalic anhydride,
wherein said solvent is one kind or two or more kinds of solvent selected from the group consisting of methanol, ethanol, propanol, isopropanol, butanol, isobutanol, secondary butanol, tertiary butanol, pentanol, isopentanol, secondary pentanol, neoptentanol, tertiary pentanol, hexanol, secondary hexanol, heptanol, secondary heptanol, octanol, 2-ethylhexanol, benzene, toluene, xylene, hexane, heptane, decane, and cyclohexane,
wherein, with respect to 1 mol of an n-equivalent olefin, 0.5n to 2n mol of the acid anhydride and 0.5n to 2n mol of urea peroxide are mixed and the mixture is reacted, and
wherein, with respect to 1 mol of the acid anhydride, 0.7 to 1.5 mol of urea peroxide is mixed.

2. The process for epoxidizing olefins according to claim 1, wherein the olefins comprise alicyclic olefins.

## Patentansprüche

1. Verfahren zur Epoxidation von Olefinen, umfassend Umsetzen eines Olefins mit einem Gemisch, welches durch Mischen eines Säureanhydrids mit einem Harnstoffperoxid in einem Lösungsmittel erhalten wird,
wobei das genannte Säureanhydrid ein Säureanhydrid oder zwei oder mehrere Säureanhydride ausgewählt aus der Gruppe bestehend aus Ameisensäureanhydrid, Essigsäureanhydrid, Propionsäureanhydrid, Bernsteinsäureanhydrid, Maleinsäureanhydrid und Phthalsäureanhydrid ist (sind),
wobei das genannte Lösungsmittel ein Lösungsmittel oder zwei oder mehrere Lösungsmittel ausgewählt aus der Gruppe bestehend aus Methanol, Ethanol, Propanol, Isopropanol, Butanol, Isobutanol, sekundäres Butanol, teritäres Butanol, Pentanol, Isopentanol, sekundäres Pentanol, Neopentanol, tertiäres Pentanol, Hexanol, sekundäres Hexanol, Heptanol, sekundäres Heptanol, Oktanol, 2-Ethylhexanol, Benzol, Toluol, Xylol, Hexan, Heptan, Dekan und Cyclohexan ist (sind),
wobei, bezogen auf 1 mol eines n-wertigen Olefins, 0,5 n bis 2 n Mol Säureanhydrid und 0,5 n bis 2 n Mol Harnstoffperoxid gemischt werden und das Gemisch umgesetzt wird und
wobei 0,7 bis 1,5 mol Harnstoffperoxid, bezogen auf 1 mol Säureanhydrid, gemischt werden.

2. Verfahren zur Epoxidation von Olefinen gemäß Anspruch 1, wobei die Olefine alicyclische Olefine umfassen.

## Revendications

1. Procédé pour l'époxydation d'oléfines, comprenant la réaction d'une oléfine avec un mélange obtenu en mélangeant un anhydride d'acide avec du peroxyde d'urée dans un solvant,
dans lequel ledit anhydride d'acide est un type ou deux ou plusieurs types d'anhydride d'acide choisis dans le groupe constitué par l'anhydride formique, l'anhydride acétique, l'anhydride propionique, l'anhydride succinique, l'anhydride maléique et l'anhydride phtalique,
dans lequel ledit solvant est un type ou deux ou plusieurs types de solvant choisis dans le groupe constitué du méthanol, de l'éthanol, du propanol, de l'isopropanol, du butanol, de l'isobutanol, du butanol secondaire, du butanol tertiaire, du pentanol, de l'isopentanol, du pentanol secondaire, du néopentanol, du pentanol tertiaire, de l'hexanol, de l'hexanol secondaire, de l'heptanol, de l'heptanol secondaire, de l'octanol, du 2-éthylhexanol, du benzène, du toluène, du xylène, de l'hexane, de l'heptane, du décane et du cyclohexane,
dans lequel, sur la base de 1 mole d'une oléfine n-équivalente, de 0,5 n à 2 n moles d'anhydride d'acide et de 0,5 n à 2 n moles de peroxyde d'urée sont mélangées et le mélange est mis à réagir, et
dans lequel, sur la base de 1 mole d'anhydride d'acide, de 0,7 à 1,5 mole de peroxyde d'urée est mélangée.

2. Procédé pour l'époxydation d'oléfines selon la revendication 1, dans lequel les oléfines comprennent des oléfines alicycliques.
